# EUROPÄISCHE PATENTSCHRIFT

(11) **EP 4 122 895 B1**
(45) Veröffentlichungstag und Bekanntmachung des Hinweises auf die Patenterteilung: **14.05.2025**
(21) Anmeldenummer: 22184456.6
(22) Anmeldetag: 12.07.2022
(51) Int. Cl.: C02F 1/52, C02F 1/48, C02F 1/72, C02F 3/34, C02F 101/10, C02F 103/02

(54) **VERFAHREN ZUR AUFBEREITUNG VON WÄSSERN, BÖDEN, SEDIMENTEN UND/ODER SCHLÄMMEN**
METHOD FOR THE TREATMENT OF WATERS, SOILS, SEDIMENTS AND / OR SLUDGES
PROCÉDÉ DE TRAITEMENT DES EAUX, DES SOLS, DES SÉDIMENTS ET/OU DES BOUES

(30) Priorität: 13.07.2021 EP 21185368
(43) Veröffentlichungstag der Anmeldung: 25.01.2023
(73) Patentinhaber: OASE GmbH, 48477 Hörstel-Riesenbeck (DE)
(72) Erfinder: MUCK, Thorsten, 49479 Ibbenbüren (DE)
(74) Vertreter: Isarpatent

(56) Entgegenhaltungen:
- EP-A1- 4 077 224
- WO-A1-01/50863
- WO-A1-2018/169395
- WO-A1-2021/144121
- WO-A1-99/58457
- DE-A1- 19 851 345
- YUAN QING ET AL: "Biosynthesis of vivianite from microbial extracellular electron transfer and environmental application", SCIENCE OF THE TOTAL ENVIRONMENT, ELSEVIER, AMSTERDAM, NL, vol. 762, 16 October 2020 (2020-10-16), XP086446735, ISSN: 0048-9697, [retrieved on 20201016], DOI: 10.1016/J.SCITOTENV.2020.143076
- WANG RU ET AL: "Fe(III) reduction and vivianite formation in activated sludge", SEPARATION AND PURIFICATION TECHNOLOGY, vol. 220, 9 March 2019 (2019-03-09) - 9 March 2019 (2019-03-09), pages 126 - 135, XP085654032, ISSN: 1383-5866, DOI: 10.1016/J.SEPPUR.2019.03.024
- HERMANS MARTIJN ET AL: "Biogeochemical impact of cable bacteria on coastal Black Sea sediment", BIOGEOSCIENCES, vol. 17, no. 23, 2 December 2020 (2020-12-02), pages 5919 - 5938, XP093007423, DOI: 10.5194/bg-17-5919-2020

## Beschreibung

Die vorliegende Erfindung betrifft ein Verfahren zur Bildung und Gewinnung von Vivianit in einem phosphorhaltigen Gewässer, Sediment und/oder Schlamm, eine Vorrichtung zur Gewinnung von Vivianit aus einem phosphorhaltigen Gewässer, Sediment und/oder Schlamm, sowie die Verwendung einer Zusammensetzung umfassend mindestens ein Erdalkaliperoxid und einer magnetischen Trennvorrichtung zur Gewinnung von Vivianit aus einem phosphorhaltigen Gewässer, Sediment und/oder Schlamm.

In Folge hoher Phosphorbelastung (Eutrophierung) bildet sich in Seen und Gewässern beschleunigt Schlamm aus partikulärem organischem Material (POM) wie abgestorbenen Algen und Pflanzenteilen, die nicht vollständig mineralisiert sind.

In Folge der Anwesenheit von Fe-Ionen kann sich hierbei in natürlicher Weise im Schlamm Vivianit (Fe(II)₃(PO₄)₂·8H₂O) bilden, welches hierbei den Phosphor binden kann, wie etwa in L. Heinrich et al., Water Research 189 (2021), 116609 beschrieben ist. Hierdurch kann eine Bindung von Phosphor in Wässern erzielt werden, und einer Eutrophierung entgegenwirkt werden. Eine gezielte Bildung von Vivianit wurde jedoch nicht erzielt.

Darüber hinaus wurde Vivianit auch in Abwässern gefunden, aus denen es auch abgetrennt werden kann, wie in T. Prot et al., Separation and Purification Technology, DOI: https://doi.org/10.1016/j.seppur.2019.05.057 beschrieben. Für Gewässer, Sedimente und Schlämme in natürlicher Umgebung ist jedoch dies aufgrund der komplexen Vorgänge im Ökosystem schwer durchführbar.

Die Dokumente DE 198 51 345 A1, WO 01/50863 A1, WO 99/58457 A1 und WO 2018/169395 A1 stellen den relevantesten Stand der Technik für den Gegenstand der vorliegenden Erfindung dar.

Durch seine Bedeutung als Grundstoff für die Herstellung von Lithiumakkumulatoren, beispielsweise für die Herstellung von Lithiumeisenphosphat, ist Vivianit jedoch auch von wirtschaftlichem Interesse, neben seiner Verwendung als Farbstoff.

Es ist somit ein Problem der vorliegenden Erfindung, eine Phosphorentfernung aus Gewässern, Schlämmen und/oder Sedimenten bereitzustellen, welche reproduzierbar gute Ergebnisse liefert und einen Wertstoff generiert.

Es wurde überraschend gefunden, dass sich durch Zugabe einer Zusammensetzung umfassend mindestens ein Erdalkaliperoxid in und/oder auf ein Gewässer, ein Sediment und/oder einen Schlamm die Vivianit-Bildung forcieren lässt, woraufhin sich Vivianit einfach magnetisch abtrennen lässt.

Ein erster Aspekt der vorliegenden Erfindung betrifft ein Verfahren zur Gewinnung von Vivianit aus einem phosphorhaltigen Gewässer, Sediment und/oder Schlamm, wobei das Gewässer anoxische Zonen aufweist und/oder anoxische Zonen erzeugt werden, umfassend:
(a) Einbringen und/oder Aufbringen einer Zusammensetzung umfassend mindestens ein Erdalkaliperoxid in und/oder auf das Gewässer, das Sediment und/oder den Schlamm;(b) Bilden von Vivianit in dem Gewässer, Sediment und/oder Schlamm; und
(c) magnetische Abtrennung des Vivianits aus dem Gewässer, Sediment und/oder Schlamm, und wobei, so nicht genügend Eisenionen vorhanden sind, Eisenionen, insbesondere Fe²⁺ - Ionen, ein- und/oder aufgebracht werden.

In einem zweiten Aspekt ist eine Vorrichtung zur Gewinnung von Vivianit aus einem phosphorhaltigen Gewässer, Sediment und/oder Schlamm offenbart, umfassend:
(i) eine Einrichtung zum Einbringen und/oder Aufbringen einer Zusammensetzung umfassend mindestens ein Erdalkaliperoxid, die dazu ausgebildet ist, die Zusammensetzung umfassend mindestens ein Erdalkaliperoxid in das Gewässer, das Sediment und/ oder den Schlamm einzubringen und/oder auf das Gewässer, das Sediment und/ oder den Schlamm aufzubringen, wobei die Einrichtung zum Einbringen und/oder Aufbringen einer Zusammensetzung umfassend mindestens ein Erdalkaliperoxid die Zusammensetzung umfassend mindestens ein Erdalkaliperoxid umfasst; und
(ii) eine magnetische Trennvorrichtung umfassend einen Magneten, die dazu ausgebildet ist, den Vivianit aus dem Gewässer, Sediment und/oder Schlamm abzutrennen.

Daneben betrifft die Erfindung die Verwendung einer Zusammensetzung umfassend mindestens ein Erdalkaliperoxid und einer magnetischen Trennvorrichtung zur Gewinnung von Vivianit aus einem phosphorhaltigen Gewässer, Sediment und/oder Schlamm.

Vorteilhafte Ausgestaltungen und Weiterbildungen ergeben sich aus den weiteren Unteransprüchen sowie aus der Beschreibung unter Bezugnahme auf die Figuren.

Die beiliegenden Figuren sollen ein weiteres Verständnis der Ausführungsformen der Erfindung vermitteln. Sie veranschaulichen Ausführungsformen und dienen im Zusammenhang mit der Beschreibung der Erklärung von Prinzipien und Konzepten der Erfindung. Andere Ausführungsformen und viele der genannten Vorteile ergeben sich im Hinblick auf die Zeichnungen. Die Elemente der Zeichnungen sind nicht notwendigerweise maßstabsgetreu zueinander gezeigt.

In den Figuren der Zeichnung sind gleiche, funktionsgleiche und gleich wirkende Elemente, Merkmale und Komponenten - sofern nichts anderes ausgeführt ist - jeweils mit denselben Bezugszeichen versehen.
FIG. 1 zeigt schematisch ein beispielhaftes erfindungsgemäßes Verfahren gemäß dem ersten Aspekt.
FIG. 2 zeigt schematisch eine beispielhafte erfindungsgemäße Vorrichtung gemäß dem zweiten Aspekt.

### Detaillierte Beschreibung der Erfindung

### Definitionen

Zunächst sollen im Kontext der vorliegenden Patentanmeldung die folgenden Begriffe wie folgt verstanden werden:
So nicht anderweitig definiert haben hierin verwendete technische und wissenschaftliche Ausdrücke dieselbe Bedeutung, wie sie von einem Fachmann auf dem Fachgebiet der Erfindung gemeinhin verstanden wird.

Kabelbakterien sind vielzellige Bakterien, die kettenförmig aneinandergereiht sind und so lange kabelartige Aggregate bilden, die über Zentimeter-Distanzen einen Elektronentransport bewerkstelligen. Sie gehören zu der Deltaproteobakterienfamilie Desulfobulbaceae, die dafür bekannt sind, dass sie sulfatreduzierende oder schwefeldisproportionierende Arten umfassen. Kabelbakterien bilden nach ihrer 16S rRNA nach derzeitigem Stand zwei Gattungen, "Candidatus Electrothrix" und "Candidatus Electronema". Mit einem "anodischen Ende" tief im Sediment und einem "kathodischen Ende" an der Phasengrenze mit Elektronenakzeptoren können sie suboxische Zonen überbrücken und in der anoxischen Zone beispielsweise Sulfid zu Sulfat oxidieren. Am "kathodischen Ende" (fungierend als Kathode) wird beispielsweise Sauerstoff reduziert, was dort zu einem pH-Wert Anstieg führt. Die Kabelbakterien ermöglichen hierbei einen Elektronentransport über lange Distanzen. Bedeutsam ist, dass diese Kabelbakterien üblicherweise nicht stabil genug sind, um aus der Sedimentoberfläche heraus zu wachsen, um an den im Wasser gelösten Sauerstoff heran zu kommen. In den stark reduzierenden Sedimenten eutropher Gewässer können

Kabelbakterien demnach nicht arbeiten. Durch ihren Elektronentransport überbrücken sie räumlich das Vorkommen von Substrat und Elektronenakzeptor. Damit sind sie anderen Organsimen, die nur direkt an der Grenzschicht der Redoxpotentiale stoffwechseln können, deutlich überlegen.

Kabelbakterien können in den eutrophierungsbeding verschlammten Gewässern demnach natürlich nicht "arbeiten", da dort der Sauerstoff als Elektronenakzeptor nicht in die obere Sedimentschicht gelangt. Mit Hilfe von Calciumperoxid können diese generell natürlichen Organismen das bekannte Kompetitionsprinzip, das auf der Basis der jeweils höchsten Redoxpotentiale der jeweiligen Elektronenakzeptoren beruht, ausschalten; Kabelbakterien schließen sich direkt mit dem jeweils höchsten vorhandenen Redoxpotential kurz und umgehen so den mikrobiologischen Wettbewerb mit Mikroorganismen, die auf den Stoffumsatz an / in der Phasengrenze beschränkt sind.

Die Oxidation von Sulfid beseitigt dessen phytotoxische Wirkung und ermöglicht submersen Wasserpflanzen somit deren Ansiedlung und Wachstum. Der Verlust der Makrophytenvegation an Gewässern ist nämlich eine bekannte und unliebsame Folge der Eutrophierung.

Kabelbakterien entkoppeln Fe(II) dadurch von einer sulfidischen Festlegung. Mobilisiertes Fe(II) reichert sich im Porenwasser an und gelangt so auch an die Phasengrenze zwischen Sediment und Wasserkörper, wo es normalerweise durch Sauerstoff zum Fe(III) oxidiert wird, welches bekannterweise Phosphat binden oder adsorbieren kann. Derartige Bindungen zwischen Phosphat und Eisen sind instabil gegenüber anoxischen Verhältnissen und lösen sich dann unter P-Freisetzung wieder auf (Eisen-Phosphor-Kreislauf).

Ein weiterer Vorteil der Kabelbakterien ist, dass sie mobil sind und sich ausrichten können, beispielsweise in Schlämmen und/oder Sedimenten sowie sogar auf Partikeln, um so eine Redoxreaktion in verschiedenen Milieus durchführen zu können.

Kabelbakterien sind beispielsweise in K.U. Kjeldsen et al, "On the evolution and physiology of cable bacteria", PNAS, 2019, www.pnas.org/cgi/doifl073/pnas.1903514116, beschrieben, wobei auf diesen Artikel bezüglich der Kabelbakterien Bezug genommen wird und dessen Inhalt in Bezug auf Kabelbakterien hiermit durch Bezugnahme aufgenommen wird.

Überraschenderweise lässt sich der natürliche Prozess der Fe- Oxidation und instabile P-Bindung durch eine vorherige Behandlung des Sedimentes mit Erdalkaliperoxid, insbesondere Calciumperoxid so umlenken, dass stattdessen eine Vivianitbildung eintritt. Es ist bekannt, dass Calciumperoxid mit Phosphat zur Apatitbildung führt. Insbesondere unter den Bedingungen einer Entkopplung der Eisen-Sulfid-Festlegung durch Kabelbakterien führt ein Anstrom von Fe(II) an das primäre Reaktionsprodukt Apatit zu einer Rekristallisation mit Vivianit als Endprodukt unter Verdrängung des Erdalkalis, insbesondere Calciums.

Im sulfidfreien, anoxischen Porenwasser kann an diesen "Impfkristallen" weiterhin das Fe(II) und weiteres Phosphat aus der Reduktion von Fe(III)-Phosphaten kristallisieren. Da Vivianit unter allen Umweltbedingungen stabil und unlöslich ist, wird so der natürliche Prozess (mit instabilen Phosphorfraktionen) in die Bildung einer nachhaltig stabilen Senke für Eisen und Phosphor umgelenkt.

Unter Gewässern bzw. Wässern werden im Sinne der vorliegenden Erfindung alle Wässer bzw. Gewässer verstanden, wie Teiche, Seen, Flüsse, Talsperren, küstennahe Gewässer, Fjorde, Brackwasserseen, Meeresbuchten, Seewasser, Aufzuchtstationen für Fische und andere Seetiere, Wasser in Speichersystemen für die Landwirtschaft oder

Trinkwasserreservoire sowie Grundwasserleiter, wobei die voranstehenden Wässer auch Böden, Sedimente und/oder Schlämme sowie Sink-und Schwebstoffe enthalten können. Unter Sedimenten werden im Sinne der vorliegenden Erfindung in Wässern befindliche Festkörper, wie z. B. in Teichen, Seen und Flüssen, verstanden. Die Schlämme können beispielsweise aus Kläranlagen, Abwasserfilter etc. stammen und/oder sich am Boden von Wässern befinden. Bevorzugte Wässer sind Süßwässer mit Sedimenten und/oder Schlammablagerungen, wie etwa Teiche, Seen, etc., welche suboxische und/oder anoxische Zonen aufweisen und schnell kippen können, welche jedoch mit den vorliegenden Verfahren effektiv behandelt werden können. Gemäß der Erfindung weisen die Gewässer bzw. Wässer anoxische Zonen auf und/oder es werden anoxische Zonen erzeugt, beispielsweise durch Entfernung von gelöstem Sauerstoff und/oder Zugabe reduzierender Mittel wie Dithionit, beispielsweise Natriumdithionit, und insbesondere weisen die anoxischen Zonen Fe-Ionen, insbesondere Fe(II) auf, was jedoch in Gewässern bzw. Wässern üblicherweise der Fall ist.

Mengenangaben im Rahmen der vorliegenden Erfindung beziehen sich auf Gew.%, soweit nicht anderweitig angegeben oder aus dem Kontext ersichtlich ist.

In einem ersten Aspekt betrifft die vorliegende Erfindung ein Verfahren zur Bildung oder Gewinnung von Vivianit in oder aus einem phosphorhaltigen Gewässer, Sediment und/oder Schlamm, umfassend:
(a) Einbringen und/oder Aufbringen einer Zusammensetzung umfassend mindestens ein Erdalkaliperoxid in und/oder auf das Gewässer, das Sediment und/oder den Schlamm;(b) Bilden von Vivianit in dem Gewässer, Sediment und/oder Schlamm; und
(c) magnetische Abtrennung des Vivianits aus dem Gewässer, Sediment und/ oder Schlamm, und wobei, so nicht genügend Eisenionen vorhanden sind, Eisenionen, insbesondere Fe²⁺ -Ionen, ein- und/oder aufgebracht werden.

Der Einfluss von Kabelbakterien auf die Fixierung von Phosphor in Gewässern ist Grundlage von Untersuchungen weltweit, wie beispielsweise in F. Sulu-Gambari et al., "Cable Bacterial Control Iron-Phosphorus Dynamics in Sediments of a Coastal Hypoxic Basin", Environmental Science & Technology, 2016, 50, 1227-1233 und M. Hermans et al., "Coupled dynamics of iron, manganese and phosphorus in brackish coastal sediments populated by cable bacteria", Limnology and Oceanography 9999, 2021, 1-21 hervorgeht, wobei aus letzterem Paper auch hervorgeht, dass der Einfluss gering ist. Vorliegend wurde überraschend gefunden, dass durch die Kombination mit einem Erdalkaliperoxid, insbesondere Calciumperoxid, die Bildung von Vivianit ermöglicht wird. Eine Bildung von Apatit, als P-reiche Vorstufe dient hier als Kondensationskern und/oder katalytisch, um mit Fe(II), das beispielsweise während oder nach Verbrauch des Erdalkaliperoxids, beispielsweise in einem dann saisonal hypoxischen Sediment, aus der Reduktion von Fe(III) freigesetzt werden kann und/oder zugeführt werden kann, reagieren kann. Entsprechend kann die Vivianit-Bildung als Stabilisationsmechanismus sukzessiv, beispielsweise nach Abschluss der Sedimentoxidation, ablaufen und somit sowohl das Fe(II) selbst, als auch das zuvor, beispielsweise mit Fe(III) gebundene, Phosphat fixieren.

Das Ein- und/oder Aufbringen der Zusammensetzung umfassend das mindestens eine Erdalkaliperoxid, insbesondere Calciumperoxid, ist nicht besonders beschränkt. Gemäß bestimmten Ausführungsformen wird die Zusammensetzung umfassend mindestens ein Erdalkaliperoxid direkt, in fester Form oder als wässrige Aufschlämmung oder Lösung, per Hand oder durch geeignete Dosiersysteme in das Wasser, den Schlamm und/oder das Sediment eingebracht und/oder auf das Wasser, den Schlamm und/oder das Sediment aufgebracht. Dies ist einfach durchführbar und ermöglicht eine

Verteilung, sodass innerhalb der Wässer, auf dem Schlamm und/oder dem Sediment annähernd gleiche Konzentrationen an Erdalkaliperoxid, insbesondere Calciumperoxid - auch über lange Zeiträume - verfügbar sind und eine gleichmäßige Bildung von Vivianit ermöglicht wird. Es wurde überraschend gefunden, dass durch das Ein- und/oder Aufbringen der Zusammensetzung umfassend das mindestens eine Erdalkaliperoxid, insbesondere Calciumperoxid, die Vivianit-Bildung begünstigt wird und Vivianit in einer Form gebildet wird, welche sich leicht aus dem Gewässer, Sediment und/oder Schlamm abtrennen lassen kann.

Die Zusammensetzung umfassend mindestens ein Erdalkaliperoxid ist nicht besonders beschränkt und umfasst gemäß bestimmten bevorzugten Ausführungsformen nur ein Erdalkaliperoxid, insbesondere Calciumperoxid. Gemäß bestimmten Ausführungsformen kann mehr als ein Erdalkaliperoxid in der Zusammensetzung enthalten sein.

Gemäß bestimmten Ausführungsformen liegt die Zusammensetzung umfassend mindestens ein Erdalkaliperoxid in fester Form vor und wird von dem zu behandelnden Gewässer umströmt. Hierdurch kann durch das mindestens eine Erdalkaliperoxid, insbesondere durch Calciumperoxid, Sauerstoff über lange Zeiträume gleichmäßig freigesetzt werden, sodass längere Einbringungsintervalle möglich sind. Gemäß bestimmten Ausführungsformen liegt die Zusammensetzung umfassend mindestens ein Erdalkaliperoxid in fester Form vor und wird mit dem Gewässer vermischt. Hierdurch kann eine homogene Verteilung im Gewässer ermöglicht werden, und somit eine gleichmäßig verteilte Vivianit-Bildung.

Gemäß bestimmten Ausführungsformen wird die Zusammensetzung umfassend mindestens ein Erdalkaliperoxid durch Zwangsmischer, Erdfräsen oder andere mechanische Dosiersysteme und/oder Methoden in das Sediment und/oder den Schlamm eingebracht bzw. auch gemäß bestimmten Ausführungsformen eingearbeitet.

Hierdurch können das Sediment und/oder der Schlamm aufgelockert und dessen bzw. deren Oberfläche für die Vivianit-Bildung vergrößert werden.

Generell kann Schritt (a) erfolgen durch direkte Dosierung der Komponenten von Hand, und/oder durch technische Einrichtungen oder Hilfsmittel wie Dosiersysteme. Das Material kann direkt in das zu behandelnde Gewässer bzw. Wasser, den Schlamm und/oder das Sediment dosiert werden, und/oder auch dadurch, dass Behältnisse das Material in fester Form enthalten und vom zu behandelnden Wasser durchströmt werden, wie zum Beispiel Filterpatronen, Fest-oder Wirbelbettreaktoren.

Geeignete Erdalkaliperoxide sind z. B. die Peroxide von Magnesium und Calcium, und deren Gemische, wobei Peroxide von Calcium und Magnesium oder deren Gemische bevorzugt eingesetzt werden. Besonders bevorzugt sind Calciumperoxide, wobei Calcium durch Magnesium in Mengenanteilen von 0,02 Gew.-% bis 50 Gew.-%, vorzugsweise bis 30 Gew.-%, bezogen auf CaO₂, ersetzt werden kann. Insbesondere bevorzugt ist Calciumperoxid, welches über lange Zeiträume Sauerstoff freisetzen kann. In handelsüblichen Produkten liegt das Erdalkaliperoxid üblicherweise im Gemisch mit dem entsprechenden Carbonat und Hydroxid vor.

Gemäß bestimmten Ausführungsformen umfasst die Zusammensetzung umfassend mindestens ein Erdalkaliperoxid weiterhin mindestens ein Erdalkalicarbonat und/oder mindestens ein Erdalkalihydroxid und/oder ein Erdalkalisulfat. Beispielweise können neben Calciumperoxid Calciumcarbonat, Calciumhydroxid und/oder Magnesiumsulfat enthalten sein. Beispielhafte Zusammensetzungen mit solchen Bestandteilen sind beispielsweise IXPER^{®}75C von Solvay. Beispielhafte geeignete Zusammensetzungen umfassend Erdalkaliperoxid sind darüber hinaus SchlixX^{®} und SchlixX^{®} plus der Söll GmbH.

Gemäß bestimmten Ausführungsformen wird das mindestens eine Erdalkaliperoxid im Gemisch mit Alkalicarbonat-Peroxyhydrat eingesetzt. Von Alkalicarbonat-Peroxyhydraten ist beim Einsatz in Wasser bei einer Dosis von 5-20 g/m³ eine unmittelbar sauerstofferhöhende Wirkung bekannt, bei der noch keine biozide Wirkung auftritt. Die Alkalicarbonat-Peroxyhydrate sind Anlagerungsprodukte von Alkalicarbonaten mit H₂O₂, wie Me₂CO₃ x H₂O₂, beispielsweise 2 Me₂CO₃ · 3 H₂O₂ (Me = Alkalimetall, beispielsweise Na, K, Rb, Cs, insbesondere Na). Sie werden auch als Alkalipercarbonate bezeichnet und sind im Handel erhältlich. Aus ökonomisch wie auch ökologischer Sicht hat sich Natriumcarbonat-Peroxyhydrat als besonders geeignet erwiesen, um niedrige Sauerstoffgehalte im Wasserkörper umgehend zu erhöhen.

Gemäß bestimmten Ausführungsformen umfasst die Zusammensetzung umfassend mindestens ein Erdalkaliperoxid weiterhin mindestens ein Alkalicarbonat-Peroxyhydrat, insbesondere Na₂CO₃ x H₂O₂, vorzugsweise 2 Na₂CO₃ · 3 H₂O₂, oder ein Gemisch aus Na₂CO₃ und H₂O₂. Gemäß bestimmten Ausführungsformen umfasst das erfindungsgemäße Verfahren weiter ein Einbringen und/oder Aufbringen von mindestens einem Alkalicarbonat-Peroxyhydrat, insbesondere Na₂CO₃ x H₂O₂, vorzugsweise 2 Na₂CO₃ · 3 H₂O₂, oder einem Gemisch aus Na₂CO₃ und H₂O₂.

Die Erdalkaliperoxide und Alkalicarbonat-Peroxyhydrate werden vorzugsweise in Mengenverhältnissen von 1 : 1 bis 1 : 0,03 eingesetzt.

Eine Steigerung der Phosphat-Fällung kann erreicht werden, wenn man dem zu behandelnden System Lanthanionen zusetzt. Diese Kombination ist besonders sinnvoll, wenn ohnehin auch eine Phosphatfällung zur Verringerung der Eutrophierung angezeigt ist. Lanthan in der aktiven Sedimentbarriere verringert den Phosphorausstrom aus dem Sediment, wenn es nach der CaO₂-Behandlung wieder reduzierend wird.

Zur Durchführung des erfindungsgemäßen Verfahrens werden die verwendeten Substanzen, d.h. Erdalkaliperoxide und ggf. Alkalicarbonat-Peroxyhydrate sowie weitere fakultative Bestandteile, gemäß bestimmten Ausführungsformen in einer Menge von 2 bis 700, bevorzugt 4 bis 500, insbesondere 5 bis 300, besonders bevorzugt 10 bis 150, g/m² Wasserfläche aufgebracht. Bei der Sediment- und/oder Schlammbehandlung kann wegen der üblicherweise höheren Menge an oxidierbaren Stoffen der Zusatz der erfindungsgemäß verwendeten Substanzen ein Vielfaches der Gewässern zuzusetzenden Menge betragen. In diesen Fällen kann eine mehrmalige Anwendung zur vermehrten Bildung von Vivianit erfolgen.

Das erfindungsgemäß eingesetzte mindestens eine Erdalkaliperoxid und weitere ggf. eingesetzte Komponenten können den zu behandelnden Systemen entweder als Einzelsubstanzen oder im Gemisch mit anderen in einem Festkörper, wässrige Lösungen oder Aufschlämmungen zugesetzt werden.

Als derartige Festkörper kommen insbesondere Silikate, wie Schichtsilikate oder Gerüstsilikate, vorzugsweise aus der Gruppe der Zeolithe und Bentonite, in Betracht. Aus anwendungstechnischen Gründen ist es besonders zweckmäßig, die in fester Form vorliegenden Materialien zu kompaktieren und z. B. als Granulate, Pellets oder Tabletten einzusetzen. Gemäß bestimmten Ausführungsformen umfasst die Zusammensetzung umfassend mindestens ein Erdalkaliperoxid daher weiterhin mindestens ein Silikat, wie Schichtsilikate oder Gerüstsilikate, vorzugsweise aus der Gruppe der Zeolithe und Bentonite.

In Abhängigkeit von der Wasserqualität und Sedimentqualität, wie Carbonatgehalt, pH-Wert, etc., kann es angebracht sein, noch weitere Verbindungen zuzusetzen, die die Wasser-bzw. Sedimentqualität erhöhen. Als solche Verbindungen können beispielsweise genannt werden Ca(OH)₂, CaO, CaCO₃, CaCl₂, Ca(NO₃)₂, CaSO₄, MgSO₄, Ca₂SiO₄, weitere analoge Magnesiumverbindungen, sowie Gemische der voranstehenden. Je nach Beschaffenheit des Rohwassers kann es erforderlich sein, das Gewässer, das Sediment und/oder den Schlamm unter Verwendung von Alkalimetall- oder Erdalkalimetallsalzen, insbesondere Oxiden, Hydroxiden, Carbonaten und/oder Hydrogencarbonaten, zu behandeln, beispielsweise um den pH-Wert zu steuern.

Gemäß bestimmten Ausführungsformen werden die erfindungsgemäß eingesetzten Verbindungen in Kombination mit einem Gemisch aus CaCO₃, CaCl₂ und/oder Ca(NO₃)₂ und ggf. Magnesiumsalzen, sowie NaHCO₃ und ggf. KHCO₃, eingesetzt, wobei CaCO₃ und CaCl₂ und/oder Ca(NO₃)₂ sowie ggf. Magnesiumsalze in einem Stoffmengenverhältnis von 0,01 : 1 bis 2 : 1 und CaCl₂ und/oder Ca(NO₃)₂ sowie ggf. Magnesiumsalze und NaHCO₃ sowie ggf. KHCO₃ in einem Stoffmengenverhältnis von 1 : 3 bis 2 : 1 vorliegen. Eine solche Mischung und ihre Eignung zur Aufbereitung von Wässern und Sedimenten ist beispielsweise in der europäischen Patentanmeldung EP 737 169 beschrieben. Gemäß bestimmten Ausführungsformen wird die Zusammensetzung umfassend mindestens ein Erdalkaliperoxid weiterhin mit einem Gemisch aus CaCO₃, CaCl₂ und/oder Ca(NO₃)₂ und ggf. Magnesiumsalzen, sowie NaHCO₃ und ggf. KHCO₃, wobei CaCO₃ und CaCl₂ und/oder Ca(NO₃)₂ sowie ggf. Magnesiumsalze in einem Stoffmengenverhältnis von 0,01 : 1 bis 2 : 1 und CaCl₂ und/oder Ca(NO₃)₂ sowie ggf. Magnesiumsalze und NaHCO₃ sowie ggf. KHCO₃ in einem Stoffmengenverhältnis von 1 : 3 bis 2 : 1 vorliegen, eingesetzt.

Das Bilden von Vivianit in dem Gewässer, Sediment und/oder Schlamm in Schritt (b) ist nicht besonders beschränkt. So Eisenionen, insbesondere Fe²⁺-Ionen, im Gewässer, Sediment und/oder Schlamm vorhanden sind, kann die Vivianit-Bildung einfach durch Ablauf von Zeit erfolgen, wobei hier vorteilhaft ist, wenn Kabelbakterien vorhanden sind, welche zudem durch eine Schwefelzyklus die Vivianit-Bildung weiter begünstigen können.

So nicht genügend Eisenionen vorhanden sind, bzw. auch um eine Eisenbildung weiter zu forcieren, werden gemäß der Erfindung Eisenionen, insbesondere Fe²⁺-Ionen, ein- und/oder aufgebracht. Gemäß bestimmten Ausführungsformen werden zusätzlich Eisenionen, insbesondere Fe²⁺-Ionen, in Schritt (b) und/oder vor und/oder nach Schritt (b), bevorzugt in Schritt (b) und/oder nach Schritt (b), eingebracht und/ oder aufgebracht. Das Einbringen und/oder Aufbringen kann parallel zum Einbringen und/oder Aufbringen einer Zusammensetzung umfassend mindestens ein Erdalkaliperoxid in und/oder auf das Gewässer, das Sediment und/oder den Schlamm erfolgen, oder auch konsekutiv, also danach, beispielsweise innerhalb von 24 h oder weniger, 12h oder weniger, oder sogar 6 h oder weniger.

Von Vorteil ist, wenn in Schritt (b) im Gewässer, Sediment und/oder Schlamm Kabelbakterien vorhanden sind. Hierzu kann ggf. auch eine Untersuchung vor Schritt (a) und/oder (b) stattfinden, um herauszufinden, ob Kabelbakterien vorhanden sind, und auch bevorzugt ob sie in ausreichender Konzentration bzw. Dichte vorhanden sind. So Kabelbakterien nicht oder nicht in ausreichender Konzentration vorhanden sind, können sie gemäß bestimmten Ausführungsformen eingebracht werden und/oder kann das Wachstum bzw. eine Vermehrung der Kabelbakterien durch geeignete Maßnahmen angeregt werden. Beispielsweise kann gemäß bestimmten Ausführungsformen bei geringer Dichte von Kabelbakterien (z.B. < 10¹ Zellen/cm² oder < 100 Zellen/m²) zunächst eine geringe Dosis an Calciumperoxid ausgebracht werden, um eine In Situ Vorkultur zu entwickeln, bevor, beispielsweise frühestens 2 Monate, 3 Monate oder 4 Monate später, eine zweite Ausbringung erfolgt.

Ebenso ist das optionale Einbringen von Kabelbakterien nicht besonders beschränkt. Beispielsweise können sie als Suspension mit Wasser als Lösungsmittel eingebracht werden. Da insbesondere bei ausreichenden Substratverhältnisse (O₂/S²⁻) die Kabelbakterien sich sehr gut vermehren können, sind die zur effektiven Animpfung erforderlichen Konzentrationen nicht sehr hoch; mit 10² bis 10⁵ Zellen/mL, reichen beispielsweise wenige Liter einer entsprechenden Vorkultur, z.B. 2, 3, 4, 5, 6, 7, 8, 9, oder 10 Liter oder mehr, für 1 ha Wasserfläche bereits aus. Höhere Startkonzentrationen oder Mengen verkürzen die Etablierungsdauer. Die Kabelbakterien sind ebenfalls nicht besonders beschränkt, und können beispielsweise auf den Gewässertyp abgestimmt sein. Beispielsweise können sie aus einer Vorkultur bereitgestellt werden, die im Labor oder im Feld, auf einer Teilfläche oder Gesamtfläche des Gewässers kultiviert werden kann. Gemäß bestimmten Ausführungsformen umfasst Schritt (b) ein Einbringen von Kabelbakterien in das Gewässer, das Sediment und/oder den Schlamm.

Das Untersuchen des Gewässers, Sediments und/oder Schlamms auf die Anwesenheit von Kabelbakterien ist ebenfalls nicht besonders beschränkt, wird jedoch vor den jeweiligen Schritten von Ein- und/oder Aufbringen in Schritt (a) und/oder dem optionalen Einbringen von Kabelbakterien durchgeführt, da das Einbringen von den Untersuchungsergebnissen abhängt, sodass auf die Untersuchungsergebnisse üblicherweise gewartet wird. Das Untersuchen kann beispielsweise durch Entnahme einer Probe umfassend Feststoffe, z.B. Schwebstoffe, Schlämme, Sedimente und mikrobiologischer Untersuchung der Probe auf Kabelbakterien erfolgen, beispielsweise durch Untersuchung mit Kabelbakterien-spezifischen Markern, z.B. mit optischen Methoden wie bei FISH (fluorescence in situ hybridization), durch Gensequenzierung von in der Probe vorhandenen Genen oder Genomen, etc. Die mikrobiologische Untersuchung ist nicht besonders beschränkt und kann beispielsweise auch diverse Vorbereitungsschritte wie etwa Reinigungs- und/oder Konzentrierungsschritte umfassen.

Wenn ausreichend Kabelbakterien in einer Probe gefunden werden, ist es nicht mehr erforderlich, das Kabelbakterien eingebracht werden. Werden jedoch im Wesentlichen keine, z.B. z.B. < 10¹ Zellen/cm² oder < 100 Zellen/m², oder keine Kabelbakterien gefunden, so sind diese einzubringen und/oder deren Wachstum ist anzuregen, wie dargelegt.

Das Einbringen und/oder Aufbringen einer Zusammensetzung umfassend mindestens ein Erdalkaliperoxid in und/oder auf das Gewässer, das Sediment und/oder den Schlamm und das optionale Einbringen von Kabelbakterien können voneinander unabhängig oder gleichzeitig durchgeführt werden. So Kabelbakterien eingebracht werden, werden diese bevorzugt jedoch derart eingebracht, dass die Kabelbakterien sofort mit der Verwertung des durch das mindestens eine Erdalkaliperoxid, insbesondere Calciumperoxid, freigesetzten Sauerstoffs und/oder anderen dadurch bereitgestellten Elektronenakzeptoren, beispielsweise das Erdalkaliperoxid, z.B. Calciumperoxid, selbst, beginnen können. Ein zeitlicher Abstand zwischen dem Einbringen der Kabelbakterien und Schritt (a) ist hierbei nicht besonders beschränkt, kann jedoch beispielsweise bis zu einer Woche, bevorzugt bis zu 3 Tage, 2 Tage, 1 Tage oder weniger, beispielsweise 12 h und weniger oder 8 h und weniger betragen.

Kabelbakterien bevorzugen ein eher pH-neutrales Milieu. Da unmittelbar nach der Ausbringung von einem Erdalkaliperoxid, insbesondere Calciumperoxid, der pH-Wert in einer obersten Schicht temporär ansteigen kann, wird bevorzugt der pH-Wert an einer Sedimentphasengrenze beobachtet, bis sich dieser auf Werte zwischen 8,7 und 7,4 normalisiert hat, beispielsweise bevor Kabelbakterien eingebracht werden.

Insbesondere Calciumperoxid kann als Produkt in, beispielsweise u.s., Zusammensetzungen so verarbeitet werden, dass mit der Einbringung kein erhöhter pH-Wert zu erwarten ist, was für die praktische Umsetzung der Erfindung vorteilhaft ist.

Die Zusammensetzung umfassend mindestens ein Erdalkaliperoxid und die Kabelbakterien, so sie eingebracht werden, werden bevorzugt an im Wesentlichen gleichen Stellen oder an derselben Stelle in das Gewässer, das Sediment und/oder den Schlamm eingebracht, um Diffusionsvorgänge zu minimieren und die Effizienz der Vivianit-Bildung zu erhöhen. Hierbei zeigt sich insbesondere der Synergismus zwischen der Sauerstofffreisetzung des mindestens einen Erdalkaliperoxids, insbesondere Calciumperoxid, und/oder anderen von Erdalkaliperoxid, z.B. Calciumperoxid, abgeleiteten Elektronenakzeptoren, und deren Verwendung durch die Kabelbakterien, welche dann weiter die Vivianit-Bildung begünstigen.

Gemäß bestimmten Ausführungsformen werden Kabelbakterien derart eingebracht, dass diese sich an einen Schlamm in einem Gewässer, Ablagerungen, Schlämme generell, Sedimente etc. anlagern können und somit eine Kontaktierung von suboxischen und/oder anoxischen Bereichen des Materials und des vom mindestens einen Erdalkaliperoxid, insbesondere Calciumperoxid, freigesetzten Sauerstoffs und/oder einem anderen davon abgeleiteten festen Elektronenakzeptor, beispielsweise das Erdalkaliperoxid, z.B. CaO₂, selbst, ermöglichen, da dort dann über Redox-Vorgänge die Vivianit-Bildung begünstigt wird.

Im erfindungsgemäßen Verfahren ist die magnetische Abtrennung des Vivianits aus dem Gewässer, Sediment und/oder Schlamm im Schritt (c) weiterhin nicht besonders beschränkt. Beispielsweise kann eine geeignete magnetische Trennvorrichtung umfassend einen Magneten in das Gewässer, das Sediment und/oder den Schlamm eingebracht werden; kann die magnetische Trennvorrichtung umfassend einen Magneten derart in die Nähe des Gewässers, Sediments und/oder Schlamms gebracht werden, dass Vivianit durch ein bereitgestelltes und/oder erzeugtes Magnetfeld aus dem Gewässer, Sediment und/oder Schlamm abgetrennt werden kann; oder kann das Gewässer, das Sediment und/oder der Schlamm auf die magnetische Trennvorrichtung umfassend einen Magneten aufgebracht und/oder in die magnetische Trennvorrichtung umfassend einen Magneten eingebracht werden.

Insbesondere die letzteren Schritte lassen sich beispielsweise bei einer Abwasserreinigung in einer Kläranlage integrieren.

Die magnetische Trennvorrichtung umfassend den Magneten ist ebenfalls nicht besonders beschränkt. Der Magnet kann beispielsweise ein Permamagnet oder ein Elektromagnet sein. Auch können mehrere Magnete vorgesehen sein. Die Trennvorrichtung kann darüber auch Halterungen, ein Gehäuse, Fördermittel wie ein Förderband oder eine Winde, etc., umfassen, und kann daran angepasst werden, ob ein Gewässer, ein Sediment oder ein Schlamm bei der Vivianit-Abtrennung zu Grunde liegt.

Das erfindungsgemäße Verfahren kann auf alle denkbaren Gewässer, Schlämme und/oder Sedimente angewandt werden. Beispielsweise kann es angewendet werden zur Vivianit-Gewinnung in Gewässersedimenten, Schlämmen und/oder Wasser/Abwasser in offenen und geschlossenen wasserhaltigen Systemen, Gewässern wie Meerwasser, Brackwasser und Süßwasser, z. B. in Talsperren, künstlichen oder natürlichen Seen, Bade-oder Fischereigewässern, Zierteichen und der Aquaristik, ferner in Prozesswässern, z.B. Kläranlagen, Abwasseraufbereitungsanlagen, Recyclinganlagen, Kühlwasseranlagen und Wärmetauscheranlagen, Abwässern chemischer Produktionsanlagen, oder in Wasser, das durch Zersetzungs- und Kondensationsprozesse (z.B. Deponiesickerwasser oder Kondensat aus thermischen Abfallverwertungsanlagen) oder durch Auslaugungsprozesse entsteht (z.B. Wasser das durch kontaminierte Böden, Gewässersedimente oder Schlämme sickert).

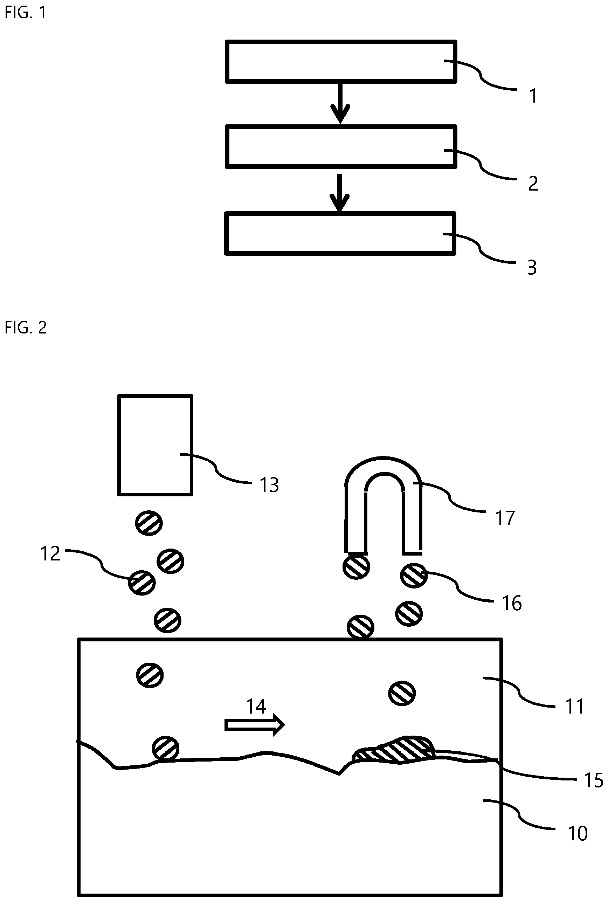

Ein beispielhaftes erfindungsgemäßes Verfahren ist schematisch in Figur 1 gezeigt. Auf ein Einbringen und/oder Aufbringen einer Zusammensetzung umfassend mindestens ein Erdalkaliperoxid 1 in und/oder auf ein Gewässer, ein Sediment und/oder einen Schlamm (Schritt (a)) folgt ein Bilden von Vivianit 2 in dem Gewässer, Sediment und/oder Schlamm (Schritt (b)), sowie eine magnetische Abtrennung des Vivianits 3 aus dem Gewässer, Sediment und/oder Schlamm (Schritt (c)).

In einem weiteren Aspekt betrifft die vorliegende Erfindung eine Vorrichtung zur Gewinnung von Vivianit aus einem phosphorhaltigen Gewässer, Sediment und/oder Schlamm, umfassend:
(i) eine Einrichtung zum Einbringen und/oder Aufbringen einer Zusammensetzung umfassend mindestens ein Erdalkaliperoxid, die dazu ausgebildet ist, die Zusammensetzung umfassend mindestens ein Erdalkaliperoxid in das Gewässer, das Sediment und/ oder den Schlamm einzubringen und/oder auf das Gewässer, das Sediment und/ oder den Schlamm aufzubringen, wobei die Einrichtung zum Einbringen und/oder Aufbringen einer Zusammensetzung umfassend mindestens ein Erdalkaliperoxid die Zusammensetzung umfassend mindestens ein Erdalkaliperoxid umfasst; und
(ii) eine magnetische Trennvorrichtung umfassend einen Magneten, die dazu ausgebildet ist, den Vivianit aus dem Gewässer, Sediment und/oder Schlamm abzutrennen.

Mit der erfindungsgemäßen Vorrichtung kann insbesondere das erfindungsgemäße Verfahren durchgeführt werden. Entsprechend sind auch bestimmte Ausführungsformen, Ausgestaltungen und Weiterbildungen des Verfahrens bei der erfindungsgemäßen Vorrichtung anwendbar, wie auch vice versa.

In der erfindungsgemäßen Vorrichtung ist die Einrichtung zum Einbringen und/oder Aufbringen einer Zusammensetzung umfassend mindestens ein Erdalkaliperoxid, die dazu ausgebildet ist, die Zusammensetzung umfassend mindestens ein Erdalkaliperoxid in das Gewässer, das Sediment und/oder den Schlamm einzubringen und/oder auf das Gewässer, das Sediment und/oder den Schlamm aufzubringen, nicht besonders beschränkt. Gemäß der Erfindung umfasst die Einrichtung zum Einbringen und/oder Aufbringen einer Zusammensetzung umfassend mindestens ein Erdalkaliperoxid die Zusammensetzung umfassend mindestens ein Erdalkaliperoxid. Die Zusammensetzung umfassend mindestens ein Erdalkaliperoxid kann hierbei gemäß bestimmten Ausführungsformen insbesondere so beschaffen sein, wie im Zusammenhang mit dem erfindungsgemäßen Verfahren beschrieben ist.

Gemäß bestimmten Ausführungsformen umfasst die Einrichtung zum Einbringen und/oder Aufbringen einer Zusammensetzung umfassend mindestens ein Erdalkaliperoxid eine Dosiereinrichtung, ausgebildet zum Dosieren der Zusammensetzung umfassend mindestens ein Erdalkaliperoxid. Die Dosiereinrichtung ist nicht besonders beschränkt. Gemäß bestimmten Ausführungsformen ist die Dosiereinrichtung derart ausgestaltet, dass sie eine zeitlich im Wesentlichen gleichbleibende oder gleichbleibende Einbringung der Zusammensetzung umfassend mindestens ein Erdalkaliperoxid ermöglicht. Hierzu kann die Dosiereinrichtung entsprechend geformt sein, etwa durch Verwendung komplexer Strukturen, und/oder entsprechende Freisetzungselemente wie etwa gesteuerte Ventile, etc., umfassen.

Daneben ist die magnetische Trennvorrichtung umfassend einen Magneten, die dazu ausgebildet ist, den Vivianit aus dem Gewässer, Sediment und/oder Schlamm abzutrennen, ebenfalls nicht besonders beschränkt. Der Magnet kann beispielsweise ein Permamagnet oder ein Elektromagnet sein. Auch können mehrere Magnete vorgesehen sein. Die Trennvorrichtung kann darüber auch Halterungen, ein Gehäuse, Fördermittel wie ein Förderband oder eine Winde, etc., umfassen, und kann daran angepasst werden, ob ein Gewässer, ein Sediment oder ein Schlamm bei der Vivianit-Abtrennung zu Grunde liegt.

Gemäß bestimmten Ausführungsformen umfasst die erfindungsgemäße Vorrichtung weiter einen Zwangsmischer und/oder eine Erdfräse ausgebildet zum Einbringen, insbesondere Einarbeiten, der Zusammensetzung umfassend mindestens ein Erdalkaliperoxid in ein Sediment und/oder einen Schlamm. Der Zwangsmischer und die Erdfräse sind nicht besonders beschränkt.

Eine beispielhafte erfindungsgemäße Vorrichtung ist schematisch in Figur 2 gezeigt.

Auf einem anoxischen Sediment 10 umfassend Kabelbakterien befindet sich ein phosphathaltiges Gewässer 11. Über eine Einrichtung zum Einbringen und/oder Aufbringen einer Zusammensetzung umfassend mindestens ein Erdalkaliperoxid 13 wird eine Zusammensetzung umfassend mindestens ein Erdalkaliperoxid 12 dosiert. Im Gewässer 11 wird Vivianit 15 gebildet, welches als Partikel 16 mittels einer magnetische Trennvorrichtung umfassend einen Magneten 17 aus dem Gewässer abgetrennt wird.

Ein weiterer Aspekt der vorliegenden Erfindung ist auf die Verwendung einer Zusammensetzung umfassend mindestens ein Erdalkaliperoxid und einer magnetischen Trennvorrichtung zur Gewinnung von Vivianit aus einem phosphorhaltigen Gewässer, Sediment und/oder Schlamm gerichtet. Die Zusammensetzung umfassend mindestens ein Erdalkaliperoxid ist hierbei insbesondere derart beschaffen, wie sie im Zusammenhang mit dem erfindungsgemäßen Verfahren und der erfindungsgemäßen Vorrichtung beschrieben ist. Ebenso ist die magnetische Trennvorrichtung insbesondere derart beschaffen, wie sie im Zusammenhang mit dem erfindungsgemäßen Verfahren und der erfindungsgemäßen Vorrichtung beschrieben ist.

Die obigen Ausgestaltungen und Weiterbildungen lassen sich, sofern sinnvoll, beliebig miteinander kombinieren. Weitere mögliche Ausgestaltungen, Weiterbildungen und Implementierungen der Erfindung umfassen auch nicht explizit genannte Kombinationen von zuvor oder im Folgenden bezüglich der Ausführungsbeispiele beschriebenen Merkmale der Erfindung. Insbesondere wird dabei der Fachmann auch Einzelaspekte als Verbesserungen oder Ergänzungen zu der jeweiligen Grundform der vorliegenden Erfindung hinzufügen.

Die Erfindung wird im Anschluss mit Bezug auf verschiedene Beispiele davon weiter im Detail erläutert. Die Erfindung ist jedoch nicht auf diese Beispiele beschränkt.

Für die folgenden Beispiele wurden die folgenden Messungen durchgeführt:
XRD: gemäß DIN EN 13925-1, -2:2003 und -3:2005, wie auch DIN EN 1330-11:2008. Für Messungen wurde nach Einfüllen die Oberfläche geglättet und gegen eine Schneide in den Strahl justiert. Gemessen wurde mit Bragg-Brentano Geometrie mit einem STOE 0/9-Diffraktometer im Reflexionsmodus, mit Strahlung einer Co- Kα1.2 Quelle mit λ~ 1,79021 Å, U = 40 kV, I = 1.35 mA

FT-IR-Spektroskopie wurde mit einem Nicolet iN10 / Nicolet iZ10 Spektrometer von Thermo Scientific im ATR-Modus mit KBr-Pellets und mit einem Jasco FT/IR-4100 Spektrometer mit einer SR-ATR Einheit mit einem Diamant-Einkristall unter konstantem Druck mit DLATGS Detektor durchgeführt.

Orthophosphat wurde gemäß DIN EN ISO 6878 gemessen. Gelöster Sauerstoff und der pH wurden mit einem HQ40 digitalem Multimeter (Hach Lange GmbH) bestimmt, und mikroskopische Aufnahmen mit einem Zeiss Axioskop 40 mit einer Canon EOS 600D mit einem EFS 18-55mm Objektiv durchgeführt.

### Referenzbeispiel 1: Phophatbindungskapazität von Calciumperoxid

Um die Phosphatbindungskapazität von CaO₂ zu erhalten, wurden 7,16 g (53 mmol) Kaliumdihydrogenphosphat zu einem 10 L Ansatz von deionisiertem Wasser gegeben. Eine anschließende Orthophosphatmessung nach DIN EN ISO 6878 ergab einen Gehalt an gelöstem Phosphat von 0,5 g/l (5,3 mmol/L). Zu dieser phosphathaltigen Lösung wurden 5,0 g eines Calciumperoxids (52 mmol reines CaO₂) zugegeben. Unmittelbar nach der Zugabe trübte sich die Lösung ein, und es wurde eine Gasentwicklung sichtbar. Nach 24 Stunden Rühren bei mittlerer Drehzahl (500 U/min) wurde die Suspension durch ein Rundfilter (MN 614, 240 mm Durchmesser) filtriert. Der Filterrückstand wurde bei 60°C über 24 Stunden getrocknet und das Filtrat einer weiteren Phosphatmessung unterzogen. Der Orthophosphatgehalt des Filtrats nach 24-stündiger Einwirkung von Calciumperoxid wurde mit 0,004 mg/L bestimmt, was einer stöchiometrischen Bindungskapazität von ca. 0,1 g Phosphat pro 1 g Calciumperoxid entspricht.

Der getrocknete Rückstand (8,1 g, bestehend aus Calciumperoxid + Phosphat; nachfolgend HA 1) wurde für die folgenden Beispiele aufbewahrt und mittels XRD und FT-IR-Spektroskopie analysiert.

Das Ausgangsmaterial Calciumperoxid (Handelsname Ixper^{®}) wurde ebenfalls untersucht und konnte als solches identifiziert werden.

### Referenzbeispiel 2: Phosphatlösungsexperimente

Um eine mögliche Wiederauflösung in einer natürlichen Umgebung zu simulieren, wurden Sedimentkerne und deren Wasserüberstand aus dem Mühlteich in der Nähe von Haldensleben entnommen und in Röhrchen gegeben. Der Test wurde mit Blindproben (ohne Zusatz von HA 1 oder Dithionit), Proben mit HA 1 (ohne Zusatz von Dithionit) (als Natriumdithionit zur Einstellung einer reduzierenden Umgebung) und Proben mit HA 1 + Dithionit durchgeführt. Alle Proben wurden doppelt durchgeführt. Da sich in den Röhrchen unterschiedlich viel Sediment befand, wurden alle Röhrchen vermessen, um die genaue Dosierung zu bestimmen. Über 3 Wochen wurden der Gehalt an Orthophosphat (nachfolgend auch PO₄), der pH-Wert und gelöster Sauerstoff (DO, dissolved oxygen) im Überstand überwacht. Die Ergebnisse sind in Tabelle 1 gezeigt.

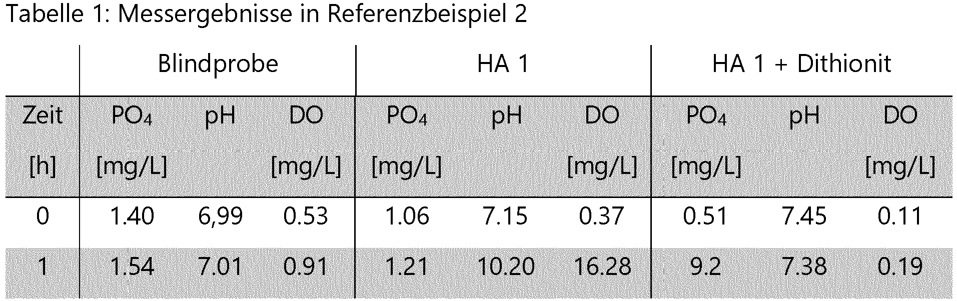

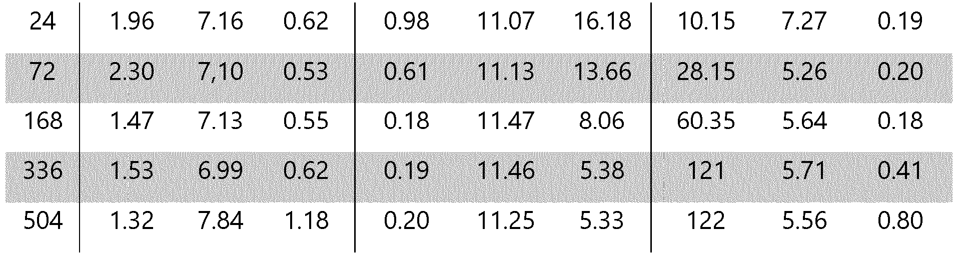

Es zeigte sich, dass unter natürlichen Bedingungen keine Phosphate im Sediment wieder aufgelöst werden. Zusätzlich bildeten sich in den Testsäulen mit Dithionit bläuliche Kristalle von Vivianit. Alle weiteren Beispiele bauen auf diesen Versuchen auf.

### Beispiel 1: Vivianitbildung und -abtrennung

Wie im Referenzbeispiel wurde HA 1 in ein reduzierendes Milieu gegeben. Verwendet wurde ein Modellsedimentkern aus Sand mit einem Überstand, der aus deionisiertem Wasser bestand, und es wurden Dithionit und Eisen als Eisen(Il)sulfat zugesetzt. Der Überstand wies die folgenden Konzentrationen pro 1 Liter entionisiertem Wasser auf:
- 4,97 g (18 mmol) Eisen(II)sulfat * 6H₂O
- 1 g Natriumdithionit

Eine Menge von 5 g (10 mmol) getrocknetem HA 1 wurde zu dieser Säule gegeben und die Säule für eine Versuchsdauer von 7 Wochen dicht verschlossen

Es bildeten sich nach einigen Wochen bläuliche Kristalle auf der oberen Sedimentschicht. Nach der gesamten Versuchsdauer von 7 Wochen wurde der Überstand durch Dekantieren unter Argonatmosphäre vom Sedimentkern getrennt. Das Sediment wurde filtriert und getrocknet. Die getrockneten blauen Kristalle wurden dann mit einem Yttrium-Magneten und mit Hilfe eines Papiers zwischen Magnet und Sediment vom Sand getrennt. Außerdem wurden die blauen Partikel (Menge 100 mg) mit Hilfe von XRD-Messungen und FT-IR-Spektroskopie untersucht und als Vivianit bestätigt.

Es zeigte sich, dass eine Umwandlung von Hydroxylapatit zu Vivianit gemäß der folgenden Formel erfolgte:

2 Ca₅(PO₄)₃(OH) + 9 FeSO₄*7 H₂O 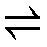

CaOH + 9 CaSO₄ * 2H₂O + 3 Fe₃(PO₄)₂*8 H₂O

Es wurden weitere Versuche durchgeführt, um die richtigen Parameter für die Vivianitbildung zu finden, wobei die Abtrennung von Vivianit in den folgenden Beispielen auch mittels Magnets erfolgte wie in Beispiel 1.

### Beispiel 2:

HA 1 (2 g, 4 mmol) wurde zu 300 mL Reinstwasser in einem Erlenmeyerkolben gegeben. Die entstehende Suspension wurde mit Argon gespült, bis der Sauerstoffgehalt unter der Nachweisgrenze von 0,02 mg/L lag (ca. 15 min). Unter Argonatmosphäre wurden 0,3 g (1,7 mmol) Natriumdithionit und 5 g (18 mmol) Eisen(II)-sulfat-Heptahydrat zugegeben. Der Kolben wurde verschlossen und die Suspension bei mittlerer Umdrehungsgeschwindigkeit (ca. 300 U/min) bei Raumtemperatur gerührt. Im Laufe von 3 Wochen färbte sich der Niederschlag grau und wurde nach 3 Wochen filtriert, gründlich mit Wasser gespült und bei 60°C über 24 h getrocknet. Während dieses Vorgangs verwandelte sich der graue Niederschlag in ein tiefes Türkis. Es wurden 1,6 g gewonnen und mittels XRD und IR-Spektroskopie analysiert, was die Vivianit-Bildung wiederum bestätigte, wie auch die Bildung der Vorstufe Hydroxylapatit sowie Gips (Verhältnis w/w Gips : Vivianit : Hydroxylapatit = 60 : 10 : 30).

### Beispiel 3

300 mL Reinstwasser wurden zu HA 1 (2 g, 4 mmol) in einem Erlenmeyerkolben gegeben. Die so entstandene Suspension wurde mit Argon gespült, bis der Sauerstoffgehalt unter der Nachweisgrenze von 0,02 mg/L lag (ca. 15 min). Unter Argonatmosphäre wurden 5 g (18 mmol) Eisen(II)-sulfat-Heptahydrat zugegeben. Der Kolben wurde verschlossen und die Suspension bei mittlerer Umdrehungsgeschwindigkeit (ca. 300 U/min) bei Raumtemperatur gerührt. Im Laufe von 3 Wochen färbte sich der Niederschlag grau, und nach 3 Wochen wurde er filtriert, gründlich mit Wasser gespült und bei 60°C über 24 h getrocknet. Es wurden 1,4 g gewonnen und mittels XRD und IR-Spektroskopie analysiert, was die Vivianit-Bildung wiederum bestätigte, wie auch die Bildung der Vorstufe Hydroxylapatit sowie Gips (Verhältnis w/w Gips : Vivianit : Hydroxylapatit = 12,7 : 30,8 : 56.5).

### Beispiel 4:

HA 1 (4 g, 8 mmol) wurde in einem Erlenmeyerkolben mit 600 mL Reinstwasser versetzt. Anschließend wurden 0,6 g (3,4 mmol) Natriumdithionit und 10 g (36 mmol) Eisen(II)-sulfat-Heptahydrat zugegeben. Der Kolben wurde verschlossen und die Suspension bei niedriger Drehzahl (ca. 90 rpm) bei Raumtemperatur geschüttelt. Im Laufe von 3 Wochen färbte sich der Niederschlag türkis, und nach 3 Wochen wurde er filtriert, gründlich mit Wasser gespült und bei 60°C über 24 h getrocknet. Während dieses Vorgangs verwandelte sich der übrige graue Niederschlag in ein tiefes Türkis. Es wurden 2,3 g gewonnen und mittels IR-Spektroskopie analysiert. Das erhaltene Gemisch zeigt die für Vivianit typischen Absorptionsbanden bei 3400 - 3300 cm⁻¹, ~1625 cm⁻¹, ~1040 cm⁻¹ und 970 cm⁻¹.

### Beispiel 5:

600 mL Reinstwasser wurden zu HA 1 (4 g, 8 mmol) in einem Erlenmeyerkolben gegeben. Die so entstandene Suspension wurde mit Argon gespült, bis der Sauerstoffgehalt unter der Nachweisgrenze von 0,02 mg/l lag (ca. 15 min). Unter Argonatmosphäre wurden 10 g (36 mmol) Eisen(II)-sulfat-Heptahydrat zugegeben. Der Kolben wurde verschlossen und die Suspension bei niedriger Drehzahl (ca. 90 U/min) bei Raumtemperatur geschüttelt. Im Laufe von 3 Wochen färbte sich der Niederschlag grau, und nach 3 Wochen wurde er filtriert, gründlich mit Wasser gespült und bei 60°C über 24 h getrocknet. Es wurden 2,1 g gewonnen und mittels IR-Spektroskopie analysiert. Das erhaltene Gemisch zeigt die typischen Absorptionsbanden von Vivianit bei 3400 - 3300 cm⁻¹, ~1625 cm⁻¹ und ~1040 cm⁻¹.

Bei der Überwachung der definierten Parameter wurde deutlich, dass unter natürlichen Bedingungen Phosphat in dem neu gebildeten Mineral HA 1 gebunden wird. Zusätzlich scheint das synthetisierte HA 1 noch mehr Phosphat zu binden. Während die Konzentrationen der Blindproben zwischen 1,5 und 2 mg/L schwanken, sinkt die Konzentration von ortho-Phosphat in den mit HA 1 behandelten Proben auf 0,2 mg/L, was durch eine unvollständige erste Reaktion und Spuren von CaO₂ gedeutet werden kann. Außerdem steigen der Sauerstoffgehalt und der pH-Wert der mit HA 1 behandelten Proben an, was auf das Vorhandensein von restlichem Peroxid und die Reaktion des Peroxids mit Wasser zu Ca(OH)₂ unter Freisetzung von O₂ zurückzuführen ist. Diese Spuren von CaO₂ sind außerdem für die beschleunigte Bildung von Hydrogensulfit (HSO₃⁻) bzw. Hydrogensulfat (HSO₄⁻) aus Natriumdithionit gemäß der folgenden chemischen Gleichung verantwortlich:

Na₂S₂O₄ + H₂O + O₂ 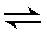

NaHSO₃ + NaHSO₄

## Patentansprüche

1. Verfahren zur Bildung und oder Gewinnung von Vivianit in bzw. aus einem phosphorhaltigen Gewässer, Sediment und/oder Schlamm, wobei das Gewässer anoxische Zonen aufweist und/oder anoxische Zonen erzeugt werden, umfassend:
(a) Einbringen und/oder Aufbringen einer Zusammensetzung umfassend mindestens ein Erdalkaliperoxid in und/oder auf das Gewässer, das Sediment und/oder den Schlamm;
(b) Bilden von Vivianit in dem Gewässer, Sediment und/oder Schlamm; und
(c) magnetische Abtrennung des Vivianits aus dem Gewässer, Sediment und/oder Schlamm, und
wobei, so nicht genügend Eisenionen vorhanden sind, Eisenionen, insbesondere Fe²⁺-Ionen, ein- und/oder aufgebracht werden.

2. Verfahren nach Anspruch 1, wobei eine Untersuchung vor Schritt (a) und/oder (b) stattfindet, um herauszufinden, ob Kabelbakterien vorhanden sind, und bevorzugt, ob sie in ausreichender Konzentration bzw. Dichte vorhanden sind, und wobei, so Kabelbakterien nicht oder nicht in ausreichender Konzentration vorhanden sind, Kabelbakterien eingebracht werden und/oder das Wachstum bzw. eine Vermehrung der Kabelbakterien durch geeignete Maßnahmen angeregt wird, bevorzugt wobei Schritt (b) ein Einbringen von Kabelbakterien in das Gewässer, das Sediment und/oder den Schlamm umfasst.

3. Verfahren nach Anspruch 1 oder 2, **dadurch gekennzeichnet, dass** als Erdalkaliperoxid ein Peroxid von Calcium, Magnesium oder eine Mischung davon eingesetzt wird.

4. Verfahren nach einem der vorgehenden Ansprüche, **dadurch gekennzeichnet, dass** die Zusammensetzung umfassend mindestens ein Erdalkaliperoxid weiterhin mindestens ein Erdalkalicarbonat und/oder mindestens ein Erdalkalihydroxid und/oder ein Erdalkalisulfat umfasst.

5. Verfahren nach einem der vorgehenden Ansprüche, **dadurch gekennzeichnet, dass** die Zusammensetzung umfassend mindestens ein Erdalkaliperoxid weiterhin mindestens ein Alkalicarbonat-Peroxyhydrat, insbesondere Na₂CO₃ x H₂O₂, vorzugsweise 2 Na₂CO₃ · 3 H₂O₂, oder ein Gemisch aus Na₂CO₃ und H₂O₂, umfasst, oder weiter umfassend ein Einbringen und/oder Aufbringen von mindestens einem Alkalicarbonat-Peroxyhydrat, insbesondere Na₂CO₃ x H₂O₂, vorzugsweise 2 Na₂CO₃ · 3 H₂O₂, oder einem Gemisch aus Na₂CO₃ und H₂O₂.

6. Verfahren nach einem der vorgehenden Ansprüche, **dadurch gekennzeichnet, dass** die Zusammensetzung umfassend mindestens ein Erdalkaliperoxid weiterhin mit einem Gemisch aus CaCO₃, CaCl₂ und/oder Ca(NO₃)₂ und ggf. Magnesiumsalze, sowie NaHCO₃ und ggf. KHCO₃, wobei CaCO₃ und CaCl₂ und/oder Ca(NO₃)₂ sowie ggf. Magnesiumsalze in einem Stoffmengenverhältnis von 0,01 : 1 bis 2 : 1 und CaCl₂ und/oder Ca(NO₃)₂ sowie ggf. Magnesiumsalze und NaHCO₃ sowie ggf. KHCO₃ in einem Stoffmengenverhältnis von 1 : 3 bis 2 : 1 vorliegen, eingesetzt wird.

7. Verfahren nach einem der vorgehenden Ansprüche, **dadurch gekennzeichnet, dass** die Zusammensetzung umfassend mindestens ein Erdalkaliperoxid weiterhin mindestens ein Silikat, wie Schichtsilikate oder Gerüstsilikate, vorzugsweise aus der Gruppe der Zeolithe und Bentonite, umfasst.

8. Verfahren nach einem der vorgehenden Ansprüche, **dadurch gekennzeichnet, dass** zusätzlich Eisenionen eingebracht und/ oder aufgebracht werden.

9. Verfahren nach einem der vorgehenden Ansprüche, **dadurch gekennzeichnet, dass** die Zusammensetzung umfassend mindestens ein Erdalkaliperoxid direkt, in fester Form oder als wässrige Aufschlämmung oder Lösung, per Hand oder durch geeignete Dosiersysteme in das Gewässer, den Schlamm und/oder das Sediment eingebracht und/oder auf das Gewässer, den Schlamm und/oder das Sediment aufgebracht wird.

10. Verfahren nach einem der vorgehenden Ansprüche, **dadurch gekennzeichnet, dass** die Zusammensetzung umfassend mindestens ein Erdalkaliperoxid in fester Form vorliegt und von dem Gewässer umströmt wird oder mit dem Gewässer vermischt wird.

11. Verfahren nach einem der Ansprüche 1 bis 9, **dadurch gekennzeichnet, dass** die Zusammensetzung umfassend mindestens ein Erdalkaliperoxid durch Zwangsmischer, Erdfräsen oder andere mechanische Dosiereinrichtungen und/oder Methoden in das Sediment und/oder den Schlamm eingebracht wird.

12. Vorrichtung zur Gewinnung von Vivianit aus einem phosphorhaltigen Gewässer, Sediment und/oder Schlamm, umfassend:
(i) eine Einrichtung zum Einbringen und/oder Aufbringen einer Zusammensetzung umfassend mindestens ein Erdalkaliperoxid, die dazu ausgebildet ist, die Zusammensetzung umfassend mindestens ein Erdalkaliperoxid in das Gewässer, das Sediment und/oder den Schlamm einzubringen und/oder auf das Gewässer, das Sediment und/oder den Schlamm aufzubringen, wobei die Einrichtung zum Einbringen und/oder Aufbringen einer Zusammensetzung umfassend mindestens ein Erdalkaliperoxid die Zusammensetzung umfassend mindestens ein Erdalkaliperoxid umfasst; und
(ii) eine magnetische Trennvorrichtung umfassend einen Magneten, die dazu ausgebildet ist, den Vivianit aus dem Gewässer, Sediment und/oder Schlamm abzutrennen.

13. Vorrichtung nach Anspruch 12, wobei die Einrichtung zum Einbringen und/oder Aufbringen einer Zusammensetzung umfassend mindestens ein Erdalkaliperoxid eine Dosiereinrichtung, ausgebildet zum Dosieren der Zusammensetzung umfassend mindestens ein Erdalkaliperoxid, umfasst.

14. Vorrichtung nach Anspruch 12 oder 13, weiter umfassend einen Zwangsmischer und/oder eine Erdfräse, ausgebildet zum Einbringen der Zusammensetzung umfassend mindestens ein Erdalkaliperoxid in ein Sediment und/oder einen Schlamm.

15. Verwendung einer Zusammensetzung umfassend mindestens ein Erdalkaliperoxid und einer magnetischen Trennvorrichtung zur Gewinnung von Vivianit aus einem phosphorhaltigen Gewässer, Sediment und/oder Schlamm.

## Claims

1. Process for forming and/or obtaining vivianite in or from a phosphorus-containing waterbody, sediment and/or sludge, respectively, comprising:
(a) introducing and/or applying a composition comprising at least one alkaline earth metal peroxide into and/or to the waterbody, the sediment and/or the sludge;
(b) forming vivianite in the waterbody, sediment and/or sludge; and
(c) magnetically removing the vivianite from the waterbody, sediment and/or sludge, and
wherein, if not enough iron ions are present, iron ions, in particular Fe²⁺ ions, are introduced and/or applied.

2. Process according to Claim 1, wherein an examination takes place before step (a) and/or (b) in order to find out whether cable bacteria are present, and preferably whether they are present in sufficient concentration or density, and wherein, if cable bacteria are not present or not present in sufficient concentration, cable bacteria are introduced and/or the growth or multiplication of the cable bacteria is stimulated by suitable measures, preferably wherein step (b) comprises introducing cable bacteria into the waterbody, the sediment and/or the sludge.

3. Process according to Claim 1 or 2, **characterized in that** alkaline earth metal peroxide used comprises a peroxide of calcium, magnesium or a mixture thereof.

4. Process according to any of the preceding claims, **characterized in that** the composition comprising at least one alkaline earth metal peroxide further comprises at least one alkaline earth metal carbonate and/or at least one alkaline earth metal hydroxide and/or an alkaline earth metal sulfate.

5. Process according to any of the preceding claims, **characterized in that** the composition comprising at least one alkaline earth metal peroxide further comprises at least one alkali metal carbonate peroxyhydrate, more particularly Na₂CO₃ x H₂O₂, preferably 2 Na₂CO₃ · 3 H₂O₂, or a mixture of Na₂CO₃ and H₂O₂, or further comprising introducing and/or applying at least one alkali metal carbonate peroxyhydrate, more particularly Na₂CO₃ × H₂O₂, preferably 2 Na₂CO₃ · 3 H₂O₂, or a mixture of Na₂CO₃ and H₂O₂.

6. Process according to any of the preceding claims, **characterized in that** the composition comprising at least one alkaline earth metal peroxide is additionally used with a mixture of CaCO₃, CaCl₂ and/or Ca(NO₃)₂ and optionally magnesium salts, and also NaHCO₃ and optionally KHCO₃, where CaCO₃ and CaCl₂ and/or Ca(NO₃)₂ and also optionally magnesium salts are present in a molar ratio of 0.01 : 1 to 2 : 1 and CaCl₂ and/or Ca(NO₃)₂ and also optionally magnesium salts and NaHCO₃ and also optionally KHCO₃ are present in a molar ratio of 1 : 3 to 2 : 1.

7. Process according to any of the preceding claims, **characterized in that** the composition comprising at least one alkaline earth metal peroxide further comprises at least one silicate, such as phyllosilicates or tectosilicates, preferably from the group of the zeolites and bentonites.

8. Process according to any of the preceding claims, **characterized in that** additionally iron ions are introduced and/or applied.

9. Process according to any of the preceding claims, **characterized in that** the composition comprising at least one alkaline earth metal peroxide is applied to the waterbody, the sludge and/or the sediment and/or introduced into the waterbody, the sludge and/or the sediment directly, in solid form or as an aqueous slurry or solution, by hand or by suitable metering systems.

10. Process according to any of the preceding claims, **characterized in that** the composition comprising at least one alkaline earth metal peroxide is present in solid form and is mixed with the waterbody or the waterbody flows around it.

11. Process according to any of Claims 1 to 9, **characterized in that** the composition comprising at least one alkaline earth metal peroxide is introduced by positive mixers, rotary tillers or other mechanical metering devices and/or methods into the sediment and/or the sludge.

12. Apparatus for obtaining vivianite from a phosphorus-containing waterbody, sediment and/or sludge, comprising:
(i) a device for introducing and/or applying a composition comprising at least one alkaline earth metal peroxide and configured to introduce the composition comprising at least one alkaline earth metal peroxide into the waterbody, the sediment and/or the sludge and/or to apply it to the waterbody, the sediment and/or the sludge; wherein the device for introducing and/or applying a composition comprising at least one alkaline earth peroxide comprises the composition comprising at least one alkaline earth peroxide; and
(ii) a magnetic separating apparatus comprising a magnet and configured to remove the vivianite from the waterbody, sediment and/or sludge.

13. Apparatus according to Claim 12, wherein the device for introducing and/or applying a composition comprising at least one alkaline earth metal peroxide comprises a metering device configured for metering the composition comprising at least one alkaline earth metal peroxide.

14. Apparatus according to Claim 12 or 13, further comprising a positive mixer and/or a rotary tiller, configured for introducing the composition comprising at least one alkaline earth metal peroxide into a sediment and/or a sludge.

15. Use of a composition comprising at least one alkaline earth metal peroxide and of a magnetic separating apparatus for obtaining vivianite from a phosphorus-containing waterbody, sediment and/or sludge.

## Revendications

1. Procédé de formation et/ou d'extraction de vivianite dans ou à partir d'une étendue d'eau, d'un sédiment et/ou d'une boue contenant du phosphore, dans lequel l'étendue d'eau présente des zones anoxiques et/ou des zones anoxiques sont créées, comprenant :
(a) l'introduction et/ou l'application d'une composition comprenant au moins un peroxyde alcalino-terreux dans et/ou sur l'étendue d'eau, le sédiment et/ou la boue ;
(b) la formation de vivianite dans l'étendue d'eau, les sédiments et/ou la boue ; et
(c) la séparation magnétique de la vivianite de l'étendue d'eau, des sédiments et/ou des boues, et
dans lequel, s'il n'y a pas suffisamment d'ions fer, des ions fer, en particulier des ions Fe²⁺, sont introduits et/ou appliqués.

2. Procédé selon la revendication 1, dans lequel une analyse a lieu avant l'étape (a) et/ou (b) pour déterminer si des bactéries câbles sont présentes, et de préférence si elles sont présentes en une concentration ou une densité suffisante, et dans lequel, si des bactéries câbles ne sont pas présentes ou ne sont pas présentes en concentration suffisante, des bactéries câbles sont introduites et/ou la croissance ou la multiplication des bactéries câbles est stimulée par des mesures appropriées, de préférence dans lequel l'étape (b) comprend une introduction de bactéries câbles dans l'étendue d'eau, le sédiment et/ou la boue.

3. Procédé selon la revendication 1 ou 2, **caractérisé en ce que** l'on utilise comme peroxyde alcalino-terreux un peroxyde de calcium, de magnésium ou un mélange de ceux-ci.

4. Procédé selon l'une des revendications précédentes, **caractérisé en ce que** la composition comprenant au moins un peroxyde d'alcalino-terreux comprend en outre au moins un carbonate alcalino-terreux et/ou au moins un hydroxyde alcalino-terreux et/ou un sulfate alcalino-terreux.

5. Procédé selon l'une des revendications précédentes, **caractérisé en ce que** la composition comprenant au moins un peroxyde alcalino-terreux comprend en outre au moins un peroxyhydrate de carbonate alcalin, notamment Na₂CO₃ x H₂O₂, de préférence 2 Na₂CO₃ · 3 H₂O₂, ou un mélange de Na₂CO₃ et de H₂O₂, ou comprenant en outre une introduction et/ou une application d'au moins un peroxyhydrate de carbonate alcalin, en particulier Na₂CO₃ x H₂O₂, de préférence 2 Na₂CO₍₃₎ . 3 H₂O₂, ou un mélange de Na₂CO₃ et de H₂O₍₂₎.

6. Procédé selon l'une des revendications précédentes, **caractérisé en ce que** la composition comprenant au moins un peroxyde alcalino-terreux est en outre utilisée avec un mélange de CaCO₃, CaCl₂ et/ou Ca(NO₃)₂ et éventuellement de sels de magnésium, ainsi que de NaHCO₃ et éventuellement KHCO₃, CaCO₃ et CaCl₂ et/ou Ca(NO₃)₂ ainsi que, le cas échéant, des sels de magnésium étant présents dans un rapport molaire de 0,01 : 1 à 2 : 1 et CaCl₂ et/ou Ca(NO₃)₂ ainsi que, le cas échéant, des sels de magnésium et NaHCO₃ ainsi que, le cas échéant, KHCO₃ étant présents dans un rapport molaire de 1 : 3 à 2 : 1.

7. Procédé selon l'une des revendications précédentes, **caractérisé en ce que** la composition comprenant au moins un peroxyde alcalino-terreux comprend en outre au moins un silicate, tel que des phyllosilicates ou des tectosilicates, de préférence choisis dans le groupe des zéolithes et des bentonites.

8. Procédé selon l'une des revendications précédentes, **caractérisé en ce que** des ions de fer sont en plus introduits et/ou appliqués.

9. Procédé selon l'une des revendications précédentes, **caractérisé en ce que** la composition comprenant au moins un peroxyde alcalino-terreux est directement, sous forme solide ou sous forme de suspension ou de solution aqueuse, manuellement ou par des systèmes de dosage appropriés, introduite dans l'étendue d'eau, la boue et/ou le sédiment et/ou appliquée sur l'étendue d'eau, la boue et/ou le sédiment.

10. Procédé selon l'une quelconque des revendications précédentes, **caractérisé en ce que** la composition comprenant au moins un peroxyde d'alcalino-terreux se présente sous forme solide et est mélangée à l'étendue d'eau ou l'étendue d'eau circule autour de celle-ci.

11. Procédé selon l'une des revendications 1 à 9, **caractérisé en ce que** la composition comprenant au moins un peroxyde d'alcalino-terreux est introduite dans le sédiment et/ou la boue par des mélangeurs à action forcés, des fraises à terre ou d'autres dispositifs et/ou méthodes de dosage mécanique.

12. Dispositif pour l'extraction de vivianite d'une étendue d'eau, d'un sédiment et/ou d'une boue contenant du phosphore, comprenant :
(i) un dispositif pour introduire et/ou appliquer une composition comprenant au moins un peroxyde alcalino-terreux, qui est conçu pour introduire la composition comprenant au moins un peroxyde alcalino-terreux dans l'étendue d'eau, le sédiment et/ou la boue et/ou pour l'appliquer sur l'étendue d'eau, le sédiment et/ou la boue, le dispositif pour introduire et/ou appliquer une composition comprenant au moins un peroxyde alcalino-terreux comprenant la composition comprenant au moins un peroxyde alcalino-terreux ; et
(ii) un dispositif de séparation magnétique comprenant un aimant, adapté pour séparer la vivianite de l'étendue d'eau, du sédiment et/ou de la boue.

13. Dispositif selon la revendication 12, dans lequel le dispositif pour l'introduction et/ou l'application d'une composition comprenant au moins un peroxyde alcalino-terreux comprend un dispositif de dosage, conçu pour doser la composition comprenant au moins un peroxyde alcalino-terreux.

14. Dispositif selon la revendication 12 ou 13, comprenant en outre un mélangeur à action forcée et/ou une fraiseuse à terre, conçu pour introduire la composition comprenant au moins un peroxyde alcalino-terreux dans un sédiment et/ou une boue.

15. Utilisation d'une composition comprenant au moins un peroxyde alcalino-terreux et un dispositif de séparation magnétique pour l'extraction de vivianite à partir d'une étendue d'eau, d'un sédiment et/ou d'une boue contenant du phosphore.
